# EUROPEAN PATENT APPLICATION

(11) **EP 2 886 636 A1**
(43) Date of publication of application: **24.06.2015**
(21) Application number: 13879572.9
(22) Date of filing: 29.05.2013
(51) Int. Cl.: C11D 17/00, A41G 1/00, C11D 9/22, C11D 9/44

(54) **ARTIFICIAL FLOWER MADE OF SOAP**

(30) Priority: 15.08.2012 JP 2012180257
(71) Applicant: Volcano Corporation Limited, Koganei-shi, Tokyo 184-0013 (JP)
(72) Inventor: HAZAWA, Manabu, Koganei-shi Tokyo 184-0013 (JP)
(74) Representative: Gill, David Alan
(86) International application number: PCT/JP2013/064857
(87) International publication number: WO 2014/027493

(57) **Abstract**

The present invention provides for an artificial flower of soap which does not lose its shape when only partly used and a flower bath or a petal bath which does not produce garbage. The petals made of a soap material constituting a flower are separately prepared one by one, and the petals are adhered with a highly hydrophilic adhesive to create the artificial flower.

## Description

### Technical Field

The present invention relates to an artificial flower made of soap or a bath additive.

### Background Art

Recently, artificial flower techniques have progressed to create a wonderful artificial flower virtually indistinguishable from the real flower. Such artificial flower techniques have been applied to produce an artificial soap flower. The artificial soap flower is not just appreciated as a flower. when no longer needing to be appreciated, the artificial soap flower is not thrown away as garbage, but can be utilized for hand-washing and other purposes as soap. Therefore, unlike a fresh flower or an artificial flower, the artificial soap flower is excellent also as an eco-product that does not produce garbage.

Patent Literature 1 discloses a technical idea of a soap material that enables fine processing of petals in preparing an artificial soap flower. This idea enables fine processing, in which petals are not molded one by one, but several petals are extracted from an integral mold and then shaped or wound to prepare petals.

However, this disclosure does not focus on a method of use and convenience provided by using soap as a material for an artificial flower, but only mentions characteristics as an eco-product that does not produce garbage as described above.

For example, in the case where an artificial flower of soap is desired to be used for hand-washing, the amount of soap will be too large if the whole artificial flower is used. In this case, one or two petals are desired to be used, but if a petal is picked off, the whole shape of the artificial flower may be lost because the artificial flower is integrally molded.

Patent Literature 2 discloses a method of preparing an artificial flower of soap which is very similar to a fresh flower and a technical idea of mixing a luminous agent with a soap material to emit beautiful light in a dark place.

However, the artificial flower using a luminous material is an existing idea, and this idea does not focus an idea on a method of use and convenience provided by using soap containing a luminous material as a material for an artificial flower, and the characteristics of soap itself are not thoroughly utilized.

On the other hand, rather than an artificial flower, a real fresh flower can be floated in a bathtub for enjoyment of a flower bath, and is commercially available as described in Non Patent Literature 1. Although such a flower bath is very attractive and wonderful, a petal bath needs time and effort to pick off a fresh flower to obtain petals or needs time and effort to recover floating fresh flowers and petals after taking a bath, and when the recovery is not perfect, the remaining flowers and petals may block a water pipe.

### Citation List

### Patent Literature

Patent Literature 1:
   Japanese Patent Laid-Open No. 5-17800
Patent Literature 2:
   Japanese Patent Laid-Open No. 2012-21246

### Non Patent Literature

Non Patent Literature 1:
URL on a rose bath: http://item.rakuten.co.jp/hana-club/fa230/

### Summary of Invention

### Technical Problem

As described above, an ecological artificial flower which does not produce garbage can be prepared by using soap as a material, but it is an object of the present invention to provide an artificial soap flower which provides an improved method of use and convenience utilizing the characteristics of soap obtained by using soap as a material.

### Solution to Problem

In order to solve the problems as described above, in the artificial flower of soap according to the present invention, petals of the artificial flower are prepared one by one from a soap material having a specific gravity of 1 or less; the artificial flower is constituted by a plurality of the petals; and a highly hydrophilic adhesive is used to fix each of the petals, as described in claim 1.

Further, in the artificial flower of soap according to the present invention, the soap material can contain a fragrance such as aroma oil as an additive, as described in claim 2.

Furthermore, in the artificial flower of soap according to the present invention, the soap material can contain as an additive a thermochromic material that changes color with temperature, as described in claim 3.

In addition, in the artificial soap flower of soap according to the present invention, bath additive can be mixed with the soap material, or only a bath additive is used to prepare petals, as described in claim 4.

### Advantageous Effects of Invention

As described above, since the petals of the artificial soap flower according to the present invention are prepared individually, the required amount of petals can be used by picking off an arbitrary number of petals without losing the shape of the artificial flower, for example, picking off one or two petals for hand-washing, or picking off three to five petals for washing a handkerchief; further, even if outer petals are picked off, the whole shape of the flower is not significantly lost because every petal is separated, and the flower can be appreciated as an artificial flower.

Further, in the artificial soap flower according to the present invention, since each petal forming the artificial flower is adhered with a highly hydrophilic adhesive, when the artificial soap flower is thrown into a bath to form a flower bath, the highly hydrophilic adhesive dissolves in hot water to allows the petals to come apart, and each petal floats on the surface of the bath and a beautiful petal bath can be enjoyed because each petal has a specific gravity of 1 or less.

In the petal bath, the petals made of soap dissolve after a while, and can form a bubble bath that is often seen in foreign films, and a feeling of bubbles can be enjoyed. As a matter of course, unlike a fresh flower or a common artificial flower, petals and garbage need not be recovered, and the bath can be drained as it is after use.

The change from the flower bath to the petal bath and further to the bubble bath as described above is a new method of use that can be achieved only by the artificial flower of soap of the present invention, the method sufficiently utilizing the characteristics of soap.

### Brief Description of Drawings

[Figure 1]Figure 1 is a schematic view illustrating a configuration of an example of the artificial flower of soap according to the present invention.
[Figure 2]Figure 2 is a schematic view illustrating parts, such as a stem and a leaf, of the artificial flower of soap according to the present invention.
[Figure 3]Figure 3 is a schematic view illustrating examples of shaping a petal of the artificial flower of soap, according to the present invention.
[Figure 4]Figure 4 is a schematic view illustrating examples of preparations of the artificial flower of soap, according to the present invention.

### Description of Embodiments

Hereinafter, the embodiments of the present invention will be described with reference to drawings. Note that, hereinafter, the scope needed to describe an object of the present invention is schematically shown; the scope needed to describe the parts corresponding to the present invention will be mainly described; and the points whose description is omitted will be based on known art.

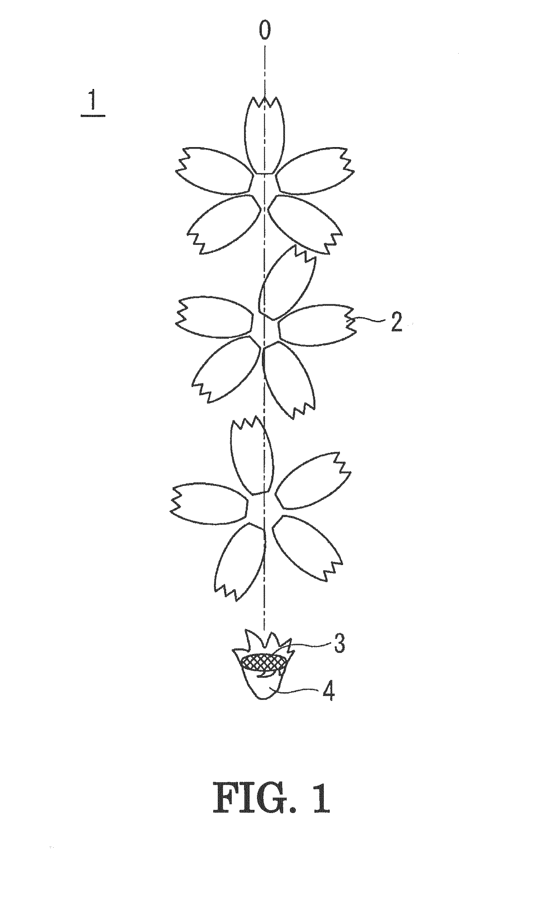

Figure 1 is a configurational schematic view of an artificial soap flower 1 according to one embodiment of the present invention. The artificial soap flower 1 comprises a plurality of petals 2, and one end of the petal 2 is adhered with a highly hydrophilic adhesive 3 that readily dissolves in water, such as methylcellulose, starch, dextrin, polyvinyl alcohol, or funori, thereby the petals being integrated into the shape of a flower.

More specifically, the following adhesives are preferably used as an adhesive to be used in the present application.
(1) A water-soluble adhesive containing at least a water-soluble polymer.
(2) A water-soluble adhesive in which the water-soluble polymer includes at least any one of a natural polymer, a semi-synthetic polymer, and a synthetic polymer.
(3) An adhesive in which the water-soluble polymer is a natural polymer. Examples of the natural polymer include starch and dextrin. Funori and a starch paste are most preferred as a water-soluble adhesive containing such a component.
(4) An adhesive in which the water-soluble polymer is a semi-synthetic polymer. In particular, a cellulose derivative is preferred as the semi-synthetic polymer. Examples of the cellulose derivative include methylcellulose, hydroxyethyl methylcellulose, and hydroxypropyl methylcellulose. Methylcellulose is most preferred.
(5) An adhesive in which the water-soluble polymer is a synthetic polymer. Examples of the synthetic polymer include polyvinyl alcohol and a copolymer thereof, sodium poly(meth)acrylate and a copolymer thereof, and polyoxyethylene and a copolymer thereof. In particular, polyvinyl alcohol is preferred.

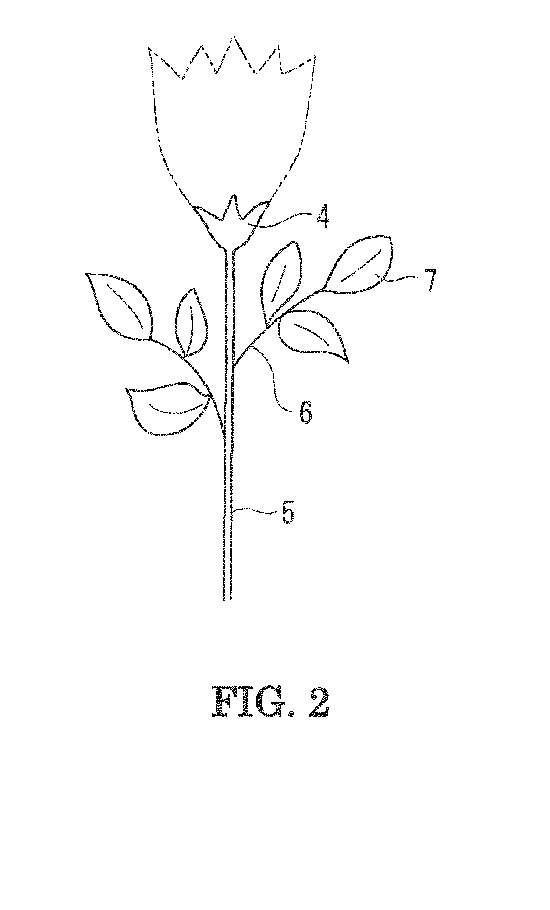

In the above description, the petals 2 may be integrated with a highly hydrophilic adhesive 3 to give an artificial flower, which may be used for a flower bath as described in Non Patent Literature 1. Alternatively, a calyx 4 made of soap may be provided in the lower part of an artificial flower as illustrated in Figure 1, and a stem 5, a branch 6, and a leaf 7 may be attached as illustrated in Figure 2.

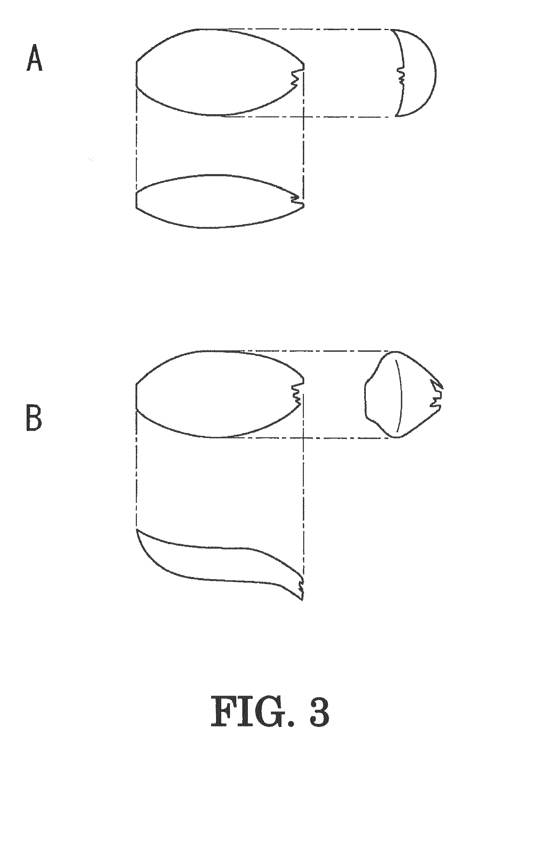

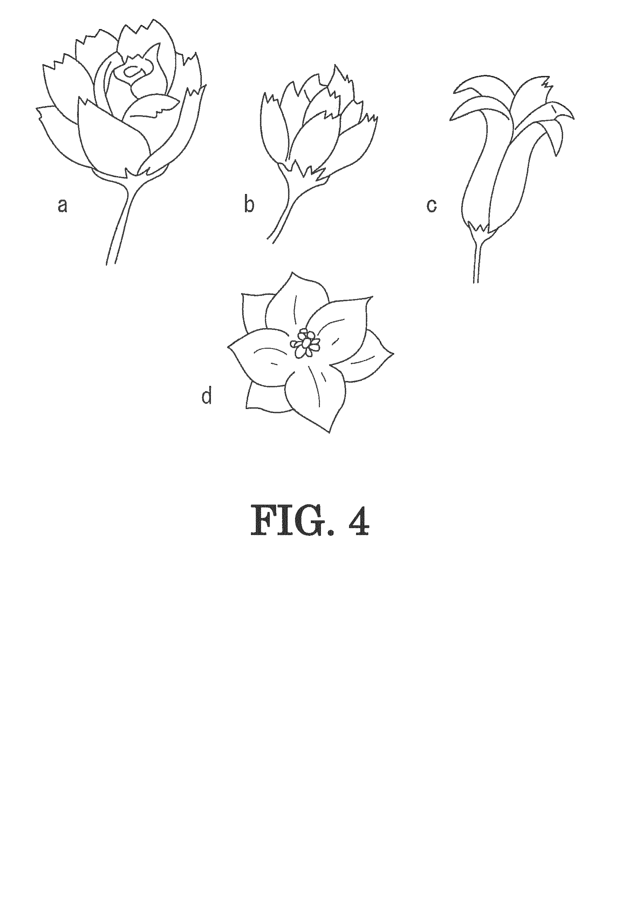

Figure 3 illustrates examples A and B for molding the petal 2, and artificial soap flowers having various shapes as illustrated in a, b, c, and d of Figure 4 can be prepared by changing the shape of the petal or the molding. Not only an imitation of a real flower but also a petal that looks more attractive when it is used in a petal bath and an artificial flower using the petal can be prepared. This is all included in the inventive concept of the present invention.

The artificial soap flower 1 according to the present invention as described above is formed in a shape as illustrated in Figure 2 for appreciation. On the other hand, when the artificial flower part is put into a bathtub, the artificial flower part can be enjoyed as a flower bath because it floats and rises to the surface of hot water since it has a specific gravity of 1 or less. The highly hydrophilic adhesive soon dissolves, and petals 2 are separately scattered all over the surface of hot water, and a petal bath can be enjoyed.

While taking the above petal 2 made of soap in hands and rubbing it or washing the body, the petal dissolves in hot water to fill a bathtub with bubbles of soap, and a bubble bath can be enjoyed. In order to further emphasize the bubbles, the petal 2 may be prepared from soap with a bath additive or from bath additive soap for bubble baths. After enjoying the bubble bath, the bath containing the petal 2 can be drained as it is in the same manner as in the case of a common soap or bath additive. It is not necessary to scoop up garbage, and a water pipe will not then become blocked.

Further, when a soap material contains aroma oil or fragrance as an additive, petals made of the resulting soap spread all over the surface of hot water, and the bath room is filled with the aroma of a flower, which can be expected to make an additional effect.

Furthermore, when a soap material contains a thermochromic material as an additive, throwing the resulting artificial soap flower into a bath instantly changes the color of the flower because the temperature in the air is clearly different from that of the hot water in the bath. This can give a great surprise effect to a user. This effect is provided by a new use of throwing the artificial soap flower of the present invention into a bath.

### Industrial Applicability

As described above, according to the invention of the present application, since petals are prepared individually and the petals are adhered with a highly hydrophilic adhesive to prepare an artificial flower, the artificial flower can be thrown into a bath to enjoy a flower bath/a petal bath/a bubble bath. These characteristics are optimum for amenity goods placed in a bathroom in a hotel. The artificial soap flower attractively decorates a bathroom as an artificial flower before use and is then used as bath soap. In addition, the flower bath/the petal bath/the bubble bath, aroma, and a change of color when the flower is thrown into a bath can be enjoyed. These can also be used also as an advertisement of a hotel.

Further, the shape of the artificial flower can be selected from amongst cherry blossoms, rose, tulip, chrysanthemum, wisteria, and the like in agreement with the seasons, thereby allowing hotel users to have the next expectation and leading to the increase in repeat customers.

Furthermore, in the same manner as in Non Patent Literature 1, the artificial soap flower can be utilized for home use or for gift-giving as a bath article with which a flower bath/a petal bath/a bubble bath can readily be enjoyed.

### Reference Signs List

1...Artificial flower of soap of the present invention, 2...Petal, 3...Hydrophilic adhesive, 4...Calyx, 5...Stem, 6...Branch, 7...Leaf

## Claims

1. An artificial flower made of a soap material, wherein petals of the artificial flower are separately prepared individually from a soap material having a specific gravity of 1 or less, and a plurality of the petals are adhered with a highly hydrophilic adhesive to each other, or to a calyx, to form the artificial flower.

2. The artificial soap flower according to claim 1, wherein the soap material comprises a fragrance as an additive.

3. The artificial soap flower according to claim 1 or 2, wherein the soap material comprises a thermochromic material as an additive.

4. The artificial soap flower according to any one of claims 1 to 3, wherein the soap material comprises a bath additive, or a bath additive is used instead of the soap material.

5. The artificial flower according to any one of claims 1 to 4, wherein the adhesive is made of a natural polymer including a cellulose derivative, funori, starch, or dextrin, or a synthetic polymer including polyvinyl alcohol or a copolymer thereof, sodium poly(meth)acrylate or a copolymer thereof, and polyoxyethylene or a copolymer thereof.

6. The artificial soap flower according to any one of claims 1 to 5, wherein the calyx is made of a soap material.
